# EUROPEAN PATENT SPECIFICATION

(11) **EP 4 526 661 B1**
(45) Date of publication and mention of the grant of the patent: **02.09.2026**
(21) Application number: 23731483.6
(22) Date of filing: 17.05.2023
(51) Int. Cl.: G01N 21/64, G01N 21/77, G01N 31/22, G01N 33/18

(54) **THIOCARBAMATE-BASED INDICATOR DETECTION OF OZONE**
THIOCARBAMATBASIERTER INDIKATOR ZUM NACHWEIS VON OZON
DÉTECTION D'OZONE PAR INDICATEUR À BASE DE THIOCARBAMATE

(30) Priority: 19.05.2022 US 202263343808 P
(43) Date of publication of application: 26.03.2025
(73) Proprietor: Hach Company, Loveland, CO 80538-8842 (US)
(72) Inventor: GREENAWALT, Angella, Nicholle, Loveland, CO 80538-8842 (US); DAS, Amit, Loveland, CO 80538-8842 (US)
(74) Representative: Sandri, Sandro
(86) International application number: PCT/US2023/067122
(87) International publication number: WO 2023/225557

(56) References cited:
- US-A1- 2021 102 930
- CHEN K ET AL: "A highly sensitive fluorescent probe for ozone based on coumarin-benzothiazole derivative", JOURNAL OF ANALYTICAL SCIENCE AND TECHNOLOGY, BIOMED CENTRAL LTD, LONDON, UK, vol. 12, no. 1, 7 April 2021 (2021-04-07), pages 1 - 8, XP021289271, DOI: 10.1186/S40543-021-00269-3
- NAGANO H ET AL: "Optimization of Fluorometric Assay for Ozone in Solution", JOURNAL OF ANALYTICAL METHODS IN CHEMISTRY 2014, 24 March 2014 (2014-03-24), pages 1 - 6, XP093063213, Retrieved from the Internet <URL:https://downloads.hindawi.com/journals/jamc/2014/190937.pdf?_gl=1*gzecrf*_ga*ODQ3NDg4NjE3LjE2ODkxNTIzMTU.*_ga_NF5QFMJT5V*MTY4OTE1MjMxNS4xLjAuMTY4OTE1MjMxNS42MC4wLjA.&_ga=2.128968718.364267984.1689152315-847488617.1689152315> [retrieved on 20230712], DOI: 10.1155/2014/190937
- GARNER A L ET AL: "Specific fluorogenic probes for ozone in biological and atmospheric samples", NATURE CHEMISTRY, vol. 1, no. 4, 31 May 2009 (2009-05-31), London, pages 316 - 321, XP093063201, ISSN: 1755-4330, Retrieved from the Internet <URL:http://www.nature.com/articles/nchem.240> [retrieved on 20230712], DOI: 10.1038/nchem.240

## Description

### TECHNICAL FIELD

This application relates generally to measuring ozone in aqueous or liquid samples, and, more particularly, to the measurement of ozone using a thiocarbamate-based indicator.

### BACKGROUND

Ensuring water quality is critical in a number of industries such as pharmaceuticals and other manufacturing fields. Additionally, ensuring water quality is critical to the health and well-being of humans, animals, and plants which are reliant on the water for survival. One element that is typically measured is ozone. Ozone may be controlled to make water safe for humans, animals, and aquatic life. Therefore, detecting the presence and concentration of ozone in water, food materials, or other liquid solutions is vital.

Article CHEN K ET AL: "A highly sensitive fluorescent probe for ozone based on coumarin-benzothiazole derivative", JOURNAL OF ANALYTICAL SCIENCE AND TECHNOLOGY, vol. 12, no. 1 (2021-04-07), DOI: 10.1186/540543-021-00269-3 discloses a detection method for monitoring ozone in living cells. A new tyoe of water-soluble fluorescent probe was syithesized by substitution reaction of 4-bromo-1-butene and hydroxycoumarin-benzothiazole derivatives, which can detect ozone in aqueous solution.

Article NAGANO H ET AL: "Optimization of Fluorometric Assay for Ozone in Solution", Journal of Analytical Methods in Chemistry 2014, 24 March 2014 (2014-03-24), DOI: 10.1155/2014/190937, discloses a method for measuring ozone with a fluoroscein derivative 1 under optimum conditions. The proble reacted with ozone quantitatively and specifically and resulted in the formation of a stable fluorescent substance.

Document US 2021/102930 A1, with its abstract, discloses a method for measuring total chlorine in a seawater sample, including: preparing a thiocarbamate-based indicator; introducing the thiocarbamate-based indicator to a seawater sample, wherein the seawater sample contains an amount of total chlorine; adding an additive to the seawater sample, wherein the additive may be potassium iodide, and wherein the additive accelerates the reaction rate between the thiocarbamate-based indicator and total chlorine and causes a change in fluorescence of the seawater sample; and measuring the amount of total chlorine in the seawater sample by measuring an intensity of the fluorescence.

### BRIEF SUMMARY

In summary, there is provided a method for measuring ozone in a sample as defined with appended independent claim 1. The dependent claims outline advantageous ways of carrying out the method.

There is also provided a measurement device configured to measure ozone in a sample, comprising the features as defined with independent claim 1.

The foregoing is a summary and thus may contain simplifications, generalizations, and omissions of detail; consequently, those skilled in the art will appreciate that the summary is illustrative only and is not intended to be in any way limiting. The invention is limited only by the scope of the appended claims.

For a better understanding of the embodiments, together with other and further features and advantages thereof, reference is made to the following description, taken in conjunction with the accompanying drawings. The scope of the invention is defined in the appended claims.

### BRIEF DESCRIPTION OF THE SEVERAL VIEWS OF THE DRAWINGS

FIG. 1 illustrates a flow diagram of an example ozone measuring system for a sample.
FIG. 2 illustrates a reaction scheme example of a thiocarbamate-based indicator for detection of ozone.
FIG. 3 illustrates an example ozone calibration curve using a thiocarbamate-based indicator from two operators.
FIG. 4A illustrates example data of ozone measurement using a thiocarbamate-based indicator.
FIG. 4B illustrates further example data of ozone measurement using a UV-Vis detection method.
FIG. 5 illustrates an example of computer circuitry.

### DETAILED DESCRIPTION

Conventional methods of ozone measurement in water may have some limitations. For example, ozone measurement may be used to determine the quality of water. High concentrations of ozone may be harmful to animals, humans, and/or plants. Accordingly, as another example, a user or entity may want the ozone in a body of water to be under a particular threshold, therefore, the user may measure the ozone to determine if the amount of ozone is under that threshold.

A standard for free and ozone measurement in water is Hach's AccuVac available from Hach Company, Loveland CO, USA (AccuVac is a registered trademark of Hach Company in the United States and other countries) which is a bleaching chemistry. These methods result in a bleaching of color in an amount proportional to the ozone concentration. The resulting color from the colorimetric reaction may be determined photometrically, for example, using a spectrophotometer. The amount of ozone may be determined by comparison to a similarly prepared blank vial. The absorbance of the sample reacted vial must be compared to the absorbance of the unreacted blank vial to determine the ozone concentration of the sample reacted vial.

However, the current analyte testing methods have limitations which are overcome by the methods and techniques as described in more detail herein. One limitation of the current techniques is that they use a bleaching chemistry not favorable to some users and measurement systems. Additionally, the traditional colorimetric methods require the preparation of a separate "blank" vial. The extra step of preparing a blank vial can introduce error to the measurement based upon individual human techniques in preparing the blank. Also, since the traditional colorimetric technique involves the bleaching of a dye, the time for preparation and time a measurement is taken, can introduce variability in the sample reading. Additionally, because the techniques include bleaching of a dye, difficulty may arise because there may not be the same volume of starting colorimetric dye in both the blank and sample vial, thereby introducing error into the determination of the amount of analyte found in the sample. This error may result in a false positive or false negative result.

According to the invention, there is provided a system and method for measuring ozone in a sample, as defined with independent claims 10,1, respectively. The sample may be drawn from a volume of liquid such as a holding tank, water source, food material source, beverage source, or the like. The sample may be free of other oxidants. In an embodiment, the method may detect ozone in concentrations in about the range of 0 to 1.5 parts per million (ppm). The method uses a fluorometric method. The indicator to give a fluorescent signal is a thiocarbamate derivative. The thiocarbamate derivative may be a derivative of 7-hydroxy coumarin. The thiocarbamate may be an umbelliferone thiocarbamate. An additive is added to the method. The additive may accelerate the completion time of a reaction. The additive is potassium iodide (KI). The fluorescence is correlated to the detection of ozone. The fluorescence intensity or change in fluorescence may be correlated to ozone in a sample. A buffer such as phosphate buffer may be added. In an embodiment, the pH of a solution may be adjusted to activate the reporter or indicator molecule. The pH may be adjusted to about pH 4.0 to about 8.5.

The illustrated example embodiments will be best understood by reference to the figures. The following description is intended only by way of example, and simply illustrates certain example embodiments.

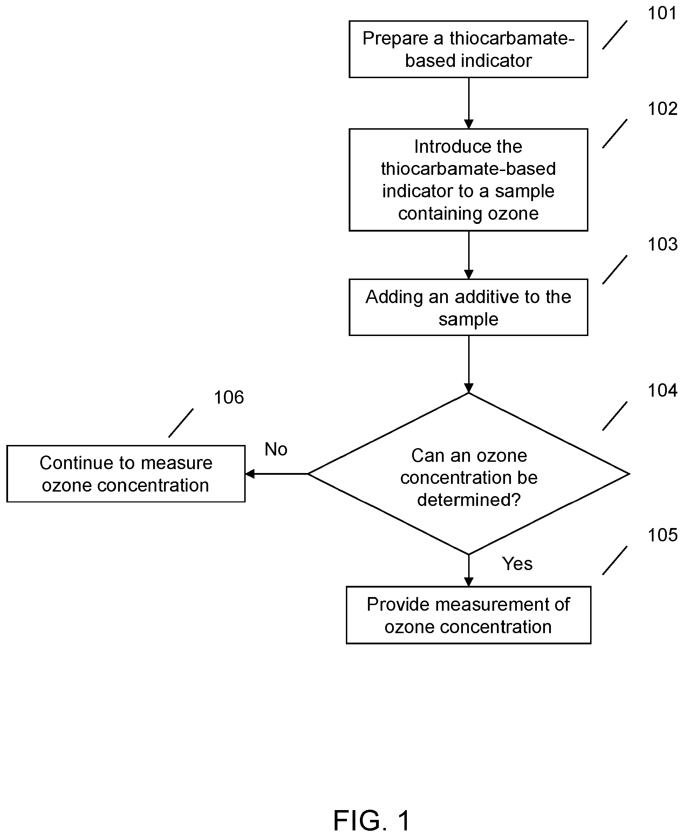

Referring to FIG. 1, a method for detection of ozone in a sample is illustrated. A thiocarbamate-based indicator is prepared. The thiocarbamate-based indicator is introduced to a sample containing ozone. An additive is added. The additive is potassium iodide (KI). The additive accelerates a reaction. The thiocarbamate-based indicator in the presence of ozone causes a change in fluorescence intensity of the thiocarbamate-based indicator. The change of fluorescent intensity is correlated to a concentration of ozone in the solution or sample.

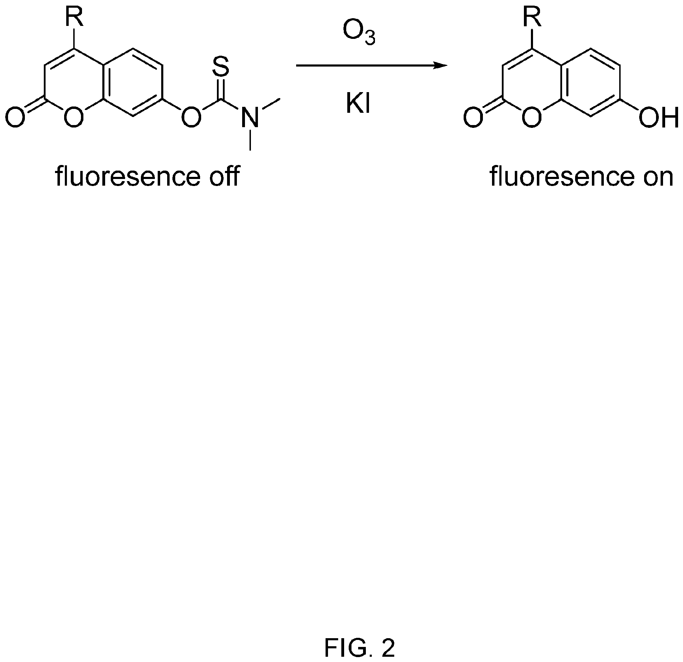

At 101, a thiocarbamate-based indicator is prepared. The thiocarbamate-based indicator may be a thiocarbamate derivative of hydroxy coumarin. In an embodiment, the thiocarbamate-based indicator may be methylumbelliferone thiocarbamate or umbelliferone thiocarbamate. Referring to FIG. 2, an example reaction of the thiocarbamate-based indicator is illustrated. In an embodiment, the thiocarbamate-based indicator may detect ozone in the range of 0 - 1.5 mg/L. The range is exemplary, a range may be determined based upon the need to control ozone to a suitable level for water treatment for example.

At 102, the thiocarbamate-based indicator is introduced into a sample. The sample contains ozone or an amount of ozone. The solution may be a sample which may include a sample from a natural body of water, a holding tank, a processing tank, a pipe, a water system, a volume of liquid for food preparation, or the like. The solution may be in a continuous flow, a standing volume of liquid, or any combination thereof. In one embodiment, the sample may be introduced to the thiocarbamate-based indicator, for example, a test chamber of the measurement device. Introduction of the sample into the measurement device may include placing or introducing the sample into a test chamber manually by a user or using a mechanical means, for example, gravity flow, a pump, pressure, fluid flow, or the like. For example, a water sample for ozone testing may be introduced to a measurement or test chamber using a pump. In an embodiment, valves or the like may control the influx and efflux of the solution into or out of the one or more chambers, if present.

Additionally, or alternatively, the measurement device may be present or introduced in a volume of the sample. The measurement device is then exposed to the volume of sample where it can perform measurements. The system may be a flow-through system in which a solution and/or reagents are automatically mixed and measured. Once the sample is in contact with the measurement system, the system may measure the ozone of the sample or a change in fluorescence of the sample, as discussed in further detail herein. In an embodiment, the measurement device may include one or more chambers in which the one or more method steps may be performed.

At 103, an additive is added to the sample. The additive is potassium iodide (KI). The additive accelerates the reaction. The additive accelerates the reaction of the thiocarbamate-based indicator and the ozone. The additive reduces the reaction time and/or accelerate the reaction of the thiocarbamate-based indicator with ozone to approximately 30 seconds. In an embodiment, the pH of the solution may be controlled. Additionally, or alternatively, ozone may be added to the solution. The ozone may be added to a sample using an ozone generator or may be present from another process. In an embodiment, the thiocarbamate-based indicator in the presence of ozone may "turn-on" the fluorescent properties of the thiocarbamate-based indicator.

In an embodiment, the pH of the solution may be adjusted. For example, the pH may be adjusted or titrated to around a pH in the range of about 4.0 to about 8.5. The thiocarbamate-based indicator concentration may be approximately 5 - 40 micromolar (µM). The indicator concentration may be adjusted based upon an ozone concentration in a sample. In an embodiment, a buffer may be added. The buffer may be a phosphate buffer. The phosphate buffer concentration may be about 75 mM. In an embodiment, the potassium iodide may be approximately 30 - 40 µM. An approximate range of detection of ozone is between 0 - 1.5 mg/L.

In an embodiment, a co-solvent may be added to the sample. The co-solvent may allow the thiocarbamate-based indicator to be more soluble in. The co-solvent may be a low molecular weight molecule. In an embodiment, the co-solvent may comprise isopropanol, ethanol, poly(ethylene glycol), poly(ethylene glycol)dimethyl ether, poly(ethylene glycol)methyl ether, or the like. In an embodiment, the co-solvent may be a mixture of the co-solvents contemplated. The co-solvent may be used at a concentration less than or equal to 10% of the sample volume.

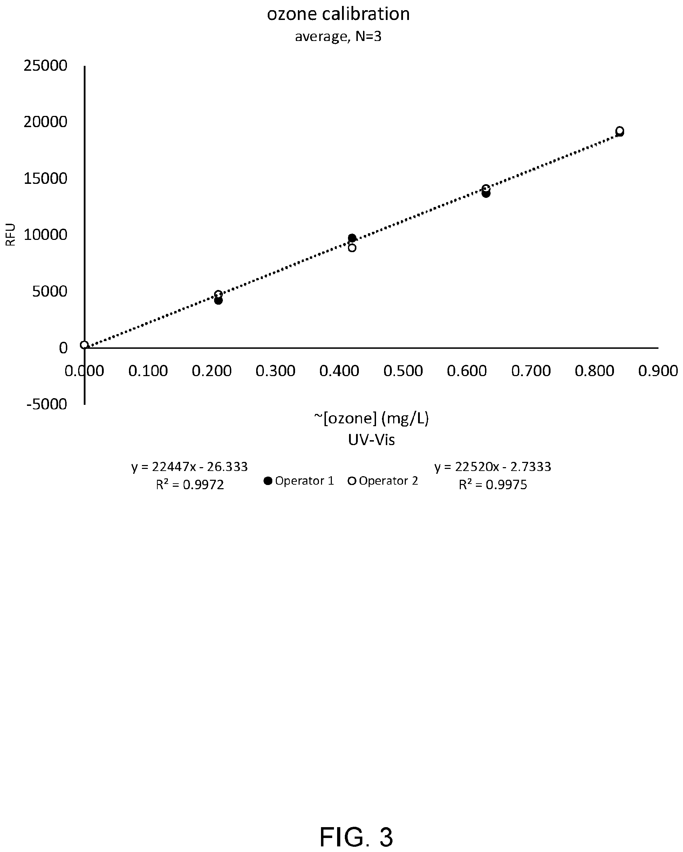

At 104, the method determines if an ozone concentration may be determined. The presence of ozone in a sample may cause an increase in fluorescence intensity of the thiocarbamate-based indicator. Examples of this increase in fluorescence intensity and dose response curves for a thiocarbamate-based indicator may be illustrated in FIG. 3. An embodiment, of relative fluorescence units (RFU) is plotted over ozone concentration (milligrams/Liter). Data from two operators is illustrated. An operator may refer to lab personnel or technician. The illustrated ozone concentration is from 0 to 0.839 mg/L. Experimental conditions may include generation of ozone with an ozone generator, at room temperature or chilling the sample to around 0 - 5 degrees Celsius, stirring the sample, pretreating the pipette tip by placing in the ozonated sample, and using a 10 cm cell to measure absorbance. These conditions are exemplary and may be altered based upon conditions. In an embodiment, the thiocarbamate-based indicator may be used at a final concentration 5 - 40 micromolar (µM).

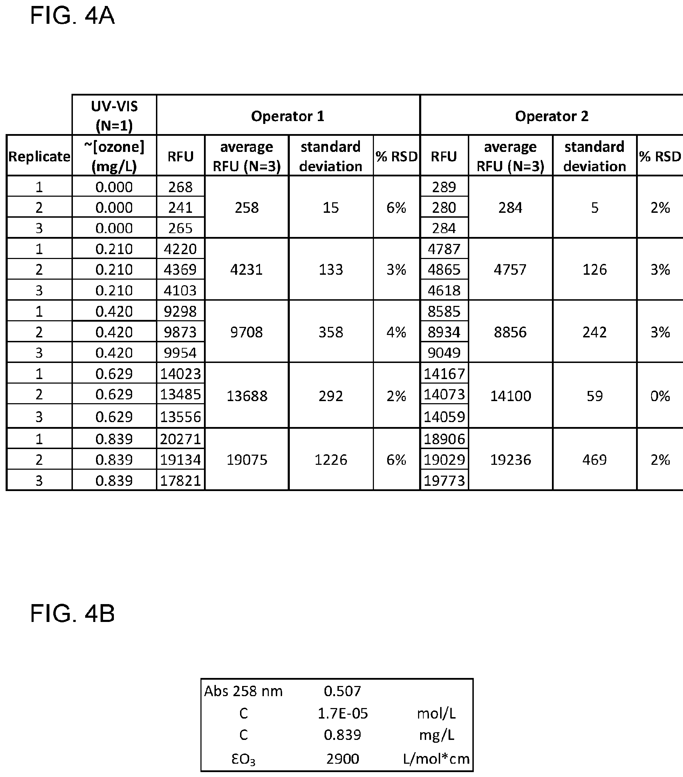

The measured absorbance is correlated to a concentration or amount of ozone in the sample or water. The correlation may be based off a UV-Vis measurement for ozone. This may be used as a reference measurement. The same aliquot may then be used in a 16 cm cell with buffer and an indicator. The sample may be diluted with deionized water depending on the ozone concentration. Measurements may be made using the Hach DR1300 FL (available from Hach Company, Loveland CO, USA) instrument to measure the RFU value or concentration versus the UV-Vis value. Operator refers to lab personnel performing the experiment. Referring to FIG. 4A, example data are illustrated using a thiocarbamate-based indicator to measure ozone concentration. Replicates by different operators are shown for different ozone concentration demonstrating the performance and accuracy of the thiocarbamate-based indicator as an ozone measurement method. In an embodiment, an excitation wavelength may be between 320 and 385 nanometers (nm). In an embodiment, an emission wavelength may be 420 - 470 nm. Referring to FIG. 4B, in an embodiment, Beer's Law is used to calculate the concentration of ozone using a 10 cm pathlength and a known molar extinction coefficient at a wavelength of 258 nm.

A phosphate buffer may be added. A solution of the thiocarbamate-based indicator may be prepared in isopropanol and added to the sample keeping the isopropanol volume of less than or equal to 10% of the sample volume. In an embodiment citrate may be added as an additive. The concentration of the citrate may be 15 mM. The citrate may prevent the formation of a metal phosphate by complexation. Potassium iodide may be added as a catalytic additive.

Therefore, the fluorescence intensity, of a solution containing ozone may be correlated to the intensity of a change in the intensity in the sample or aqueous solution. Fluorescence curves may be generated for a range of concentrations, for different thiocarbamate-based indicators, for any different condition that may affect absorption or fluorescence values (e.g., temperature, sample content, turbidity, viscosity, measurement apparatus, aqueous sample chamber, etc.), or the like.

Alternatively, or additionally, ozone measurement may be at periodic intervals set by the user or preprogrammed frequencies in the device. Measurement by a device allows for real time data with very little human involvement in the measurement process. Cleaning of the fluorometric chamber may be required at an unspecified time interval. A programmed calibration curve may be entered into the device.

A chamber, vessel, cell, chamber, or the like may contain a sample, at least one thiocarbamate-based indicator, and associated reagents such as buffers and/or additives. A device may contain one or more bottles of reagents which contain necessary reagents. The reagents contained in the one or more bottles may be pump fed or gravity fed. The flow of the reagents may be metered to ensure proper volume delivery to the measurement cell. The sample may be fed through a pressured inlet, a vessel, or the like. The sample may be introduced into the measurement chamber by a pump or gravity fed. The sampling device may be in series or parallel to an aqueous flow. The device may have a system to ensure proper mixing of the aqueous sample, thiocarbamate-based indicator, and related reagents.

The fluorescent intensity or ozone concentration may be an output upon a device in the form of a display, printing, storage, audio, haptic feedback, or the like. Alternatively, or additionally, the output may be sent to another device through wired, wireless, fiber optic, Bluetooth^{®}, near field communication, or the like. An embodiment may use an alarm to warn of a measurement or concentration outside acceptable levels. An embodiment may use a system to shut down water output or shunt water from sources with unacceptable levels of an analyte. For example, an analyte measuring device may use a relay coupled to an electrically actuated valve, or the like.

At 106, if a concentration of ozone cannot be determined, the system may continue to measure ozone, changes in fluorescence intensity and/or fluorescence intensity. Additionally, or alternatively, the system may output an alarm, log an event, or the like.

If a concentration of ozone can be determined, the system provides a measurement of ozone concentration at 105. The system may connect to a communication network. The system may alert a user or a network. This alert may occur whether an ozone measurement is determined or not. An alert may be in a form of audio, visual, data, storing the data to a memory device, sending the output through a connected or wireless system, printing the output or the like. The system may log information such as the measurement location, a corrective action, geographical location, time, date, number of measurement cycles, or the like. The alert or log may be automated, meaning the system may automatically output whether a correction was required or not. The system may also have associated alarms, limits, or predetermined thresholds. For example, if an ozone concentration reaches a threshold. Alarms or logs may be analyzed in real-time, stored for later use, or any combination thereof.

The various embodiments described herein thus represent a technical improvement to conventional ozone measurement techniques. Using the techniques as described herein, an embodiment may use a thiocarbamate-based indicator to measure ozone in solution. This is in contrast to methodology with limitations mentioned above. Such techniques provide a faster and more accurate method for measuring ozone in an aqueous or liquid solution. The various embodiments described herein thus represent a technical improvement to precise ozone measurement in a sample. Using the techniques as described herein, an embodiment may use a method and device to measure ozone concentration. This is in contrast to conventional methods with limitations mentioned above.

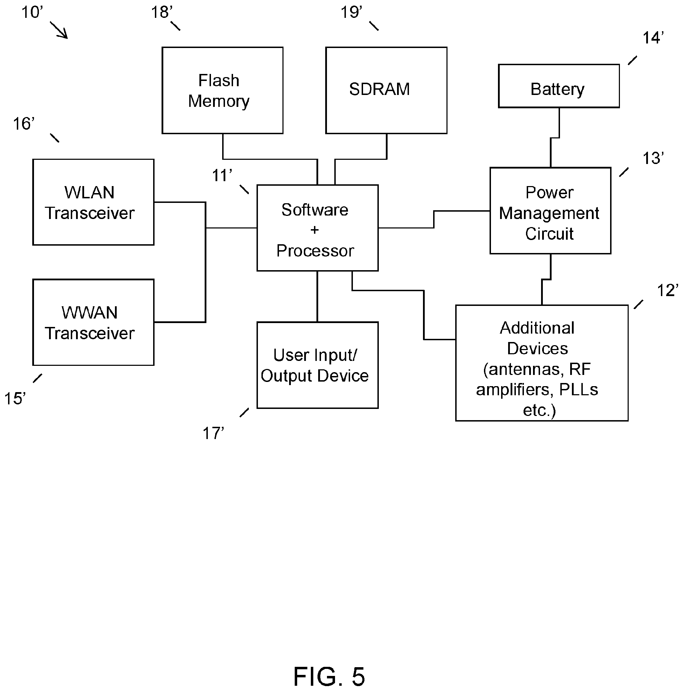

While various other circuits, circuitry or components may be utilized in information handling devices, with regard to an instrument for ozone measurement in according to any one of the various embodiments described herein, an example is illustrated in FIG. 5. Device circuitry 10' may include a measurement system on a chip design found, for example, a particular computing platform (e.g., mobile computing, desktop computing, etc.) Software and processor(s) are combined in a single chip 11'. Processors comprise internal arithmetic units, registers, cache memory, busses, I/O ports, etc., as is well known in the art. Internal busses and the like depend on different vendors, but essentially all the peripheral devices (12') may attach to a single chip 11'. The circuitry 10' combines the processor, memory control, and I/O controller hub all into a single chip 11'. Also, systems 10' of this type do not typically use SATA or PCI or LPC. Common interfaces, for example, include SDIO and I2C.

There are power management chip(s) 13', e.g., a battery management unit, BMU, which manage power as supplied, for example, via a rechargeable battery 14', which may be recharged by a connection to a power source (not shown). In at least one design, a single chip, such as 11', is used to supply BIOS like functionality and DRAM memory.

System 10' typically includes one or more of a WWAN transceiver 15' and a WLAN transceiver 16' for connecting to various networks, such as telecommunications networks and wireless Internet devices, e.g., access points. Additionally, devices 12' are commonly included, e.g., a transmit and receive antenna, oscillators, PLLs, etc. System 10' includes input/output devices 17' for data input and display/rendering (e.g., a computing location located away from the single beam system that is easily accessible by a user). System 10' also typically includes various memory devices, for example flash memory 18' and SDRAM 19'.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an embodiment to perform ozone measurement of a sample.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and products according to various example embodiments. It will be understood that the actions and functionality may be implemented at least in part by program instructions. These program instructions may be provided to a processor of a device, e.g., a handheld measurement device, or other programmable data processing device to produce a machine, such that the instructions, which execute via a processor of the device, implement the functions/acts specified.

It is noted that the values provided herein are to be construed to include equivalent values as indicated by use of the term "about." The equivalent values will be evident to those having ordinary skill in the art, but at the least include values obtained by ordinary rounding of the last significant digit.

This disclosure has been presented for purposes of illustration and description but is not intended to be exhaustive or limiting. Many modifications and variations will be apparent to those of ordinary skill in the art, limited only by the scope of the appended claims.
The example embodiments were chosen and described in order to explain principles and practical application, and to enable others of ordinary skill in the art to understand the disclosure for various embodiments with various modifications as are suited to the particular use contemplated, limited only by the scope of the appended claims.

## Claims

1. A method for measuring ozone in a sample, comprising:
(a) preparing (101) a thiocarbamate-based indicator;
(b) introducing (102) the thiocarbamate-based indicator to a sample, wherein the sample contains an amount of ozone and the introducing causes a change in fluorescence of the solution;
(c) adding (103) potassium iodide to the sample, wherein the potassium iodide accelerates the reaction rate between the thiocarbamate-based indicator and ozone and causes a change in fluorescence of the sample;
(d) measuring a change in intensity of the fluorescence of the sample; and
(e) by a processor (11'), measuring (104) the amount of ozone in the sample based on the change in intensity of the fluorescence.

2. The method of claim 1, wherein the thiocarbamate-based indicator comprises a thiocarbamate derivative of hydroxyl coumarin.

3. The method of claim 2, wherein the thiocarbamate-based derivative is a derivative of 7-hydroxy-coumarin.

4. The method of any one of the preceding claims, further comprising titrating the sample to a pH in the range of about 4.0 to about 8.5.

5. The method of any one of the preceding claims, further comprising adding a phosphate buffer.

6. The method of any one of the preceding claims, wherein the amount of ozone is in the range of 0 - 1.5 mg/L.

7. The method of any one of the preceding claims, wherein the sample comprises water from a beverage material system.

8. The method of any one of the preceding claims, further comprising addition of a co-solvent.

9. The method of claim 8, wherein the co-solvent is selected from the group consisting of: isopropanol, ethanol, poly(ethylene glycol), poly(ethylene glycol)dimethyl ether, and poly(ethylene glycol)methyl ether.

10. A measurement device configured to measure ozone in a sample, comprising:
a thiocarbamate-based indicator and at least one measurement chamber; mechanical means for introducing the thiocarbamate-based indicator into the at least one measurement chamber of the device;
mechanical means for introducing a potassium iodide (KI) additive into the at least one measurement chamber of the device;
means for measuring a change in intensity of the fluorescence of the sample; a processor; and
a memory storing instructions executable by the processor to carry out a method according to steps (b)-(e) of claim 1.

## Patentansprüche

1. Verfahren zur Messung von Ozon in einer Probe, umfassend:
(a) Herstellen (101) eines Indikators auf Thiocarbamat-Basis;
(b) Einbringen (102) des Indikators auf Thiocarbamat-Basis in eine Probe, wobei die Probe eine bestimmte Menge an Ozon enthält und das Einbringen eine Änderung der Fluoreszenz der Lösung bewirkt;
(c) Beschleunigen der Reaktionsgeschwindigkeit zwischen dem Indikator auf Thiocarbamatbasis und Ozon, wodurch eine Änderung der Fluoreszenz der Probe bewirkt wird;
(d) Messen einer Änderung der Intensität der Fluoreszenz der Probe; und
(e) Messen (104) der Ozonmenge in der Probe durch einen Prozessor (11') auf der Grundlage der Messung (104) der Ozonmenge in der Probe.

2. Verfahren nach Anspruch 1, wobei der Indikator auf Thiocarbamatbasis ein Thiocarbamatderivat von Hydroxycumarin umfasst.

3. Verfahren nach Anspruch 2, wobei das Derivat auf Thiocarbamatbasis ein Derivat von 7-Hydroxycumarin ist.

4. Verfahren nach einem der vorstehenden Ansprüche, das ferner das Titrieren der Probe auf einen pH-Wert im Bereich von etwa 4,0 bis etwa 8,5 umfasst.

5. Verfahren nach einem der vorstehenden Ansprüche, das ferner das Hinzufügen eines Phosphatpuffers umfasst.

6. Verfahren nach einem der vorstehenden Ansprüche, wobei die Ozonmenge im Bereich von 0 bis 1,5 mg/l liegt.

7. Verfahren nach einem der vorstehenden Ansprüche, wobei die Probe Wasser aus einem Getränkematerialsystem umfasst.

8. Verfahren nach einem der vorstehenden Ansprüche, das ferner die Zugabe eines Hilfslösungsmittels umfasst.

9. Verfahren nach Anspruch 8, wobei das Hilfslösungsmittel aus der Gruppe ausgewählt wird, bestehend aus: Isopropanol, Ethanol, Poly(ethylenglykol), Poly(ethylenglykol)dimethylether und Poly(ethylenglykol)methylether.

10. Messvorrichtung, die zur Messung von Ozon in einer Probe ausgelegt ist, umfassend:
einen Indikator auf Thiocarbamat-Basis und mindestens eine Messkammer;
mechanische Mittel zum Einbringen des Indikators auf Thiocarbamat-Basis in die mindestens eine Messkammer der Vorrichtung;
Mechanische Mittel zum Einbringen eines Kaliumiodid (KI)-Additivs in die mindestens eine Messkammer der Vorrichtung;
Mittel zum Messen einer Änderung der Fluoreszenzintensität der Probe;
einen Prozessor; und einen Speicher, der Befehle speichert, die vom Prozessor ausgeführt werden können, um ein Verfahren gemäß den Schritten (b) bis (e) des Anspruchs 1 durchzuführen.

## Revendications

1. Procédé de mesure de l'ozone dans un échantillon, comprenant :
(a) la préparation (101) d'un indicateur à base de thiocarbamate ;
(b) l'introduction (102) de l'indicateur à base de thiocarbamate dans un échantillon, dans lequel l'échantillon contient une quantité d'ozone et l'introduction provoque un changement de la fluorescence de la solution ;
(c) l'ajout (103) d'iodure de potassium à l'échantillon, dans lequel l'iodure de potassium accélère la vitesse de réaction entre l'indicateur à base de thiocarbamate et l'ozone et provoque un changement de la fluorescence de l'échantillon ;
(d) la mesure d'un changement d'intensité de la fluorescence de l'échantillon ; et
(e) par un processeur (11'), la mesure (104) de la quantité d'ozone dans l'échantillon sur la base du changement d'intensité de la fluorescence.

2. Procédé selon la revendication 1, dans lequel l'indicateur à base de thiocarbamate comprend un dérivé thiocarbamate d'hydroxycoumarine.

3. Procédé selon la revendication 2, dans lequel le dérivé à base de thiocarbamate est un dérivé de 7-hydroxycoumarine.

4. Procédé selon l'une quelconque des revendications précédentes, comprenant en outre le titrage de l'échantillon à un pH compris dans la plage allant d'environ 4,0 à environ 8,5.

5. Procédé selon l'une quelconque des revendications précédentes, comprenant en outre l'ajout d'un tampon phosphate.

6. Procédé selon l'une quelconque des revendications précédentes, dans lequel la quantité d'ozone est comprise dans la plage allant de 0 à 1,5 mg/L.

7. Procédé selon l'une quelconque des revendications précédentes, dans lequel l'échantillon comprend de l'eau provenant d'un système de matériaux pour boissons.

8. Procédé selon l'une quelconque des revendications précédentes, comprenant en outre l'ajout d'un co-solvant.

9. Procédé selon la revendication 8, dans lequel le co-solvant est choisi dans le groupe constitué par : l'isopropanol, l'éthanol, le poly(éthylène glycol), l'éther diméthylique de poly(éthylène glycol) et l'éther méthylique de poly(éthylène glycol).

10. Dispositif de mesure configuré pour mesurer l'ozone dans un échantillon, comprenant :
un indicateur à base de thiocarbamate et au moins une chambre de mesure ;
des moyens mécaniques pour introduire l'indicateur à base de thiocarbamate dans la au moins une chambre de mesure du dispositif ;
des moyens mécaniques pour introduire un additif d'iodure de potassium (KI) dans ladite au moins une chambre de mesure du dispositif
des moyens pour mesurer un changement d'intensité de la fluorescence de l'échantillon ;
un processeur ; et
une mémoire stockant des instructions exécutables par le processeur pour mettre en oeuvre un procédé selon les étapes (b) à (e) de la revendication 1.
